# EUROPEAN PATENT APPLICATION

(11) **EP 4 640 230 A1**
(43) Date of publication of application: **29.10.2025**
(21) Application number: 23905486.9
(22) Date of filing: 31.10.2023
(51) Int. Cl.: A61K 36/9064, A61K 36/8994, A61K 36/8945, A61K 36/62, A61K 36/484, A61K 36/48, A61K 36/346, A61K 36/284, A61K 36/258, A61K 36/076, A61P 31/14, A61P 29/00, A61P 25/24, A61P 25/22, A61P 25/20, A61P 25/00, A61P 11/00

(54) **APPLICATION OF SHENLING BAIZHU IN PREPARATION OF DRUG FOR TREATING PSYCHONEUROLOGICAL SYMPTOMS OF RECOVERED COVID-19 PATIENTS**

(30) Priority: 21.12.2022 CN 202211651746
(71) Applicant: Beijing Redsun Pharmaceutical Co., Ltd., Beijing 100020 (CN)
(72) Inventor: LIN, Deliang, Beijing 100020 (CN)
(74) Representative: Novagraaf Technologies
(86) International application number: PCT/CN2023/128825
(87) International publication number: WO 2024/131315

(57) **Abstract**

An application of Shenling Baizhu in the preparation of a medicine for treating neuropsychiatric symptoms of recovered COVID-19 patients.

## Description

### Cross-Reference to Related Application

The present application claims priority on the basis of Chinese Patent Application No. 202211651746.X, filed with the China State Intellectual Property Office on 21 December 2022, which is incorporated herein by reference in its entirety.

### Technical Field

The present disclosure belongs to the fields of medicine and traditional Chinese medicine, and particularly relates to use of Shenling Baizhu for treating neuropsychiatric symptoms of recovered COVID-19 patients.

### Background

Shenling Baizhu San comes from "Taiping Huimin Hejiju Fang" of the Song Dynasty. It is a representative prescription of "reinforcing earth to strengthen metal (invigorating spleen for benefiting lung)". In the prescription, ginseng, baizhu *(Macrocephalae Rhizoma),* and poria cocos can strengthen the spleen, benefit the lung, and eliminate dampness, and are main medicines; Chinese yam, lotus seed, hyacinth beans, and semen coicis can strengthen the spleen, excrete dampness, and relieve diarrhea, and are minister medicines; fructus amomi can invigorate the spleen and eliminate dampness, platycodon grandiflorus can disperse the lung and expel wind and carry the medicines upward, and fructus amomi and platycodon grandiflorus are all adjuvant medicines; and stir-fried licorice is used as an envoy medicine to strengthen the spleen and harmonize the stomach. The whole prescription nourishes the spleen and lung, excretes dampness, and relieves diarrhea. It is often used for lung and spleen and stomach diseases. Symptoms include fatigue, loss of appetite, loose stools, etc. The Shenling Baizhu formulation used clinically includes pills, powders, granules, and capsules.

The outbreak of Coronavirus Disease 2019 (COVID-19, also referred to below simply as "novel coronavirus infection") has been a global pandemic. According to the latest real-time statistics from the World Health Organization, as of 30 August 2022, the global cumulative number of confirmed cases of COVID-19 infection has reached 599 million, with a cumulative number of deaths of 6.467 million. Although the vast majority of infected people can meet the criteria for clinical cure, and after the acute infection period, the viral nucleic acid test has turned negative, they continue to experience a series of sequelae, including fatigue, cough, shortness of breath, poor appetite, low fever, night sweats, constipation or diarrhea, chest tightness, palpitations, chronic bleeding, insomnia, anxiety or depression, sensory disorders, movement disorders, etc.; among them, more than 20% of people are in a state of anxiety or depression 6 months after acute infection, and 26% of people have insomnia (Huang C, et al. 6-month consequences of COVID-19 in patients discharged from hospital: a cohort study[J]. Lancet. 2021;397(10270): 220-232). The described sequelae greatly affect the quality of life of patients who have recovered from COVID-19 infection, especially neuropsychiatric symptoms such as insomnia, anxiety, and depression, which cause the psychological damage from COVID-19 infection persists long-term. Therefore, providing medicines to relieve and improve neuropsychiatric symptoms for clinically cured COVID-19 patients will help them resume normal daily and work activities as soon as possible.

### Summary

In view of the shortcomings of the prior art, the present disclosure provides a new medical use of traditional Chinese medicine famous prescription Shenling Baizhu.

In order to achieve the described technical effect, the present disclosure adopts the following technical solution:
Use of Shenling Baizhu in the preparation of a medicine for treating neuropsychiatric symptoms of recovered COVID-19 patients; the raw material composition of the Shenling Baizhu comprises: ginseng, *rhizoma atractylodis macrocephalae* stir-fried with bran, poria cocos, Chinese yam, lotus seed, fried white hyacinth beans, coix seed, fructus amomi, platycodon grandiflorus, and liquorice.

Optionally, the neuropsychiatric symptoms of recovered COVID-19 patients comprise at least one of insomnia, anxiety, and depression.

Optionally, the medicine is a clinically acceptable formulation.

Optionally, the medicine is selected from a soup, a pill, a powder, a granule, a tablet, or a capsule.

The raw materials of the Shenling Baizhu comprise the following medicinal materials in parts by weight:
100 parts of ginseng, 100 parts of *rhizoma atractylodis macrocephalae* stir-fried with bran, 100 parts of poria cocos, 100 parts of Chinese yam, 50 parts of lotus seed, 75 parts of fried white hyacinth beans, 50 parts of coix seeds stir-fried with bran, 50 parts of fructus amomi, 50 parts of platycodon grandiflorus, and 100 parts of liquorice.

In the present description, the "parts by weight" or "parts" is not a weight or a mass unit, and merely represents a relative weight (mass) ratio relationship between the respective components. Depending on the actual situation, 1 part by weight can be 1 g, 4 g, 5 g, 10 g, 1 kg, or any other desired weight (mass).

By means of clinical observation, the Shenling Baizhu granule can effectively treat neuropsychiatric symptoms of recovered COVID-19 patients, so that the patients can resume normal daily and work activities as soon as possible.

### Brief Description of the Drawings

The present disclosure will be further described below with reference to the accompanying drawings.
Fig. 1 shows a sample table of self-rating anxiety scale (SAS).
Fig. 2 shows a sample table of self-rating depression scale (SDS).

### Detailed Description of the Embodiments

The 2019 novel coronavirus (2019-nCoV) not only causes serious damage to the respiratory system of infected patients, but also has a huge impact on the patient's whole body biological system, causing persistent and long-term sequelae to patients, greatly affecting their physical health and quality of life. The inventor conducted a clinical observation trial on the effect of a Shenling Baizhu formulation on improving the neuropsychiatric symptoms of recovered COVID-19 patients in four hospitals in Hubei Tongshan County People's Hospital, Sinopharm Dongfeng General Hospital, Xianning Central Hospital, and Yangxin County People's Hospital. After nearly a month of intervention treatment, it was surprisingly found that the patients' neuropsychiatric sequelae such as anxiety and depression were significantly alleviated.

The present disclosure is described below with reference to specific examples. It should be understood by a person skilled in the art that these examples are only intended to illustrate the present disclosure, and do not limit the scope of the present disclosure in any way.

The test methods in the following examples are all conventional methods if no special description is made. Medicinal materials, reagent materials, etc. used in the following examples are all commercially available products if no special description is made.

### EXAMPLE 1 Intervention of Shenling Baizhu granule on neuropsychiatric symptoms of recovered COVID-19 patients

This example was carried out in Hubei Tongshan County People's Hospital, Sinopharm Dongfeng General Hospital, Xianning Central Hospital, and Yangxin County People's Hospital from September 2021 to July 2022.

### 1. Case distribution

A total of 313 subjects were enrolled in the study, and 313 completed the study, accounting for 100% of the enrolled cases. After excluding 8 cases whose ages were out of the range of 18-80 years old, 305 cases were included in the analysis set.

### 2. General information of the case

There were 166 males (54.43%) and 139 females (45.57%); there were 303 Han people (99.34%); the average age was 46.34±14.09 years old, with the youngest being 2 years old and the oldest being 84 years old. The average weight was 63.41±7.51 (kg).

### 3. Vital signs

All physical signs such as body temperature, respiration, blood pressure, and heart rate were basically normal at enrollment, and no obvious abnormalities were found in any physical signs during the study.

### 4. Dosing regimen

During the study, the average number of days of medication was 28.09±0.88 days, the shortest number of days of medication was 25 days, the longest number of days of medication was 32 days, and the median number of days of medication was 28 days. The day before the subject started taking the medication was recorded as day 0, the day of starting medication was recorded as day 1, and so on, until day 28.

The specific dosage regimen was as follows: Shenling Baizhu Granule, 3 times a day, 1 bag each time (3 g/bag), for 28 consecutive days. The Shenling Baizhu Granule is produced by Beijing Handian Pharmaceutical Co., Ltd., with batch numbers 211032, 211134, and 211232.

### 5. Efficacy evaluation index

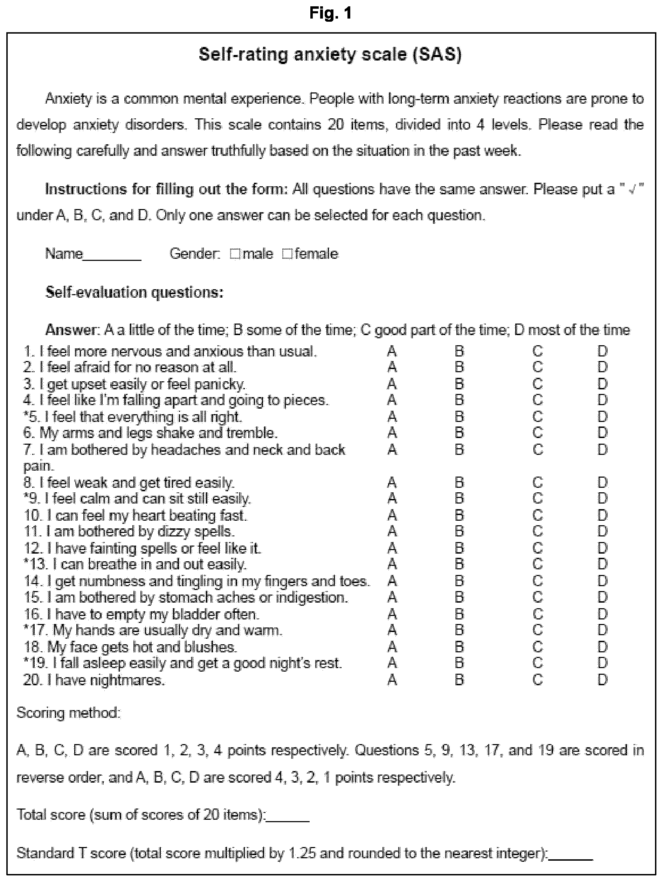

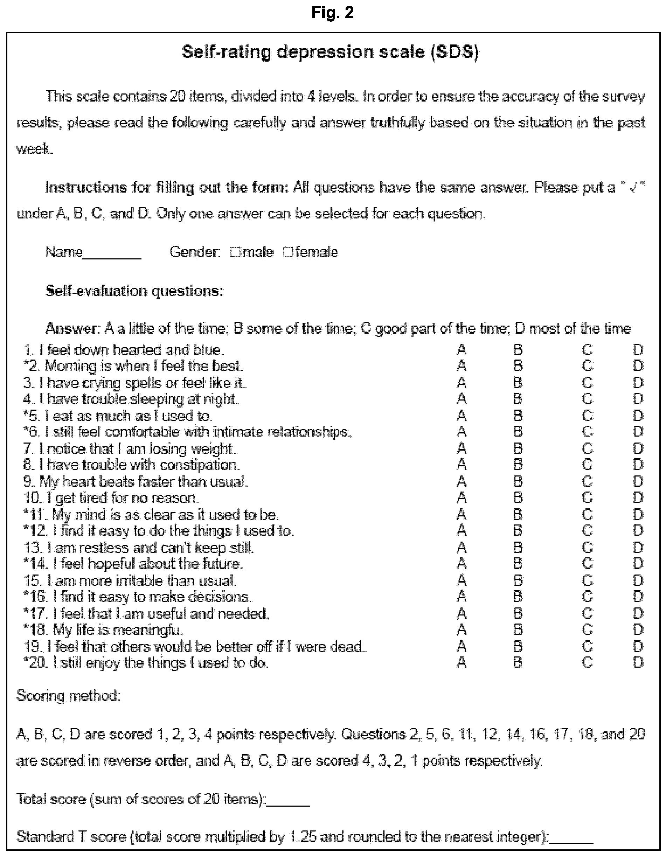

SAS score (Self-rating anxiety scale, see Fig. 1) and SDS score (Self-rating depression scale, see Fig. 2) were measured.

### 6. Data acquisition

All enrolled subjects visited the hospital on days 0, 14, and 28 to collect symptoms and score records. The data on day 0 was recorded as "Visit 1", the data on day 14 was recorded as "Visit 2", and the data on day 28 was recorded as "Visit 3". The three groups of data were statistically analyzed.

### 7. Statistical analysis

Statistical analysis was performed using SAS 9.4 (or above) statistical analysis software.

All statistical tests were two-sided, and the test statistics and their corresponding P values were given. P < 0.05 was considered to be statistically significant.

Quantitative data were described using sample size (n), mean, standard deviation, minimum, median, maximum, upper quartile (Q1), lower quartile (Q3), and 95% confidence interval (95% CI). Intergroup differences were analyzed using Student's t test. If the influence of covariates was considered, analysis of covariance (ANCOVA) was used. For time series data, the median survival time was described and the Kaplan-Meier curve was drawn. The Log-rank test was used to compare the difference in median survival time between the two groups. If the influence of the center or other factors was considered, Cox Proportional-Hazards Model was used.

Qualitative data describe the number of cases and percentages of each category. The chi-square test and Fisher's exact probability method were used to compare the nominal data among the treatment groups; the Wilcoxon rank sum test was used to compare the ordinal data among the treatment groups. If the influence of the center or other factors was taken into account, the CMH chi-square test or Logistic regression was used.

### 8. Intervention efficacy

### 8.1 SAS scale (self-rating anxiety scale) score results

The results are shown in Tables 1 and 2.

**Table 1 Measured values of SAS scale score**

| | **Visit 1** | **Visit 2** | **Visit 3** | **Statistics** | **P value** | **Test method** |
|---|---|---|---|---|---|---|
| SAS scale total score | | | | | | Mixed effect model |
| N (Nmiss) | 304(1) | 305(0) | 303(2) | 476.33 | <.0001 | |
| Mean±SD | 58.63±14.07 | 41.48±13.6 | 31.2±9.43 | Visit 1VS2 | <.0001 | |
| Median (Q1, Q3) | 53.5(46, 67) | 39(30, 50) | 26(25, 33) | Visit 1VS3 | <.0001 | |
| Min, Max | 38, 100 | 25, 86 | 25, 61 | Visit 2VS3 | <.0001 | |

**Table 2 Difference of SAS scale scores**

| | **Visit 2-Visit 1 difference** | **Visit 3-Visit 1 difference** |
|---|---|---|
| The difference between the total SAS score after treatment and the baseline | | |
| N (Nmiss) | 304 (1) | 303 (2) |
| Mean±SD | -17.21±12.45 | -27.44±15.94 |
| Median (Q1,Q3) | -17 (-23.5, -9) | -24 (-38, -18) |
| Min, Max | -63, 18 | -75, 16 |
| Statistics | -21606 | -22428 |
| P value | <0.0001 | <0.0001 |
| Test method | paired rank test | paired rank test |

Through intervention treatment, the average score of Visit 2 during the study decreased by 17.21±12.45 points compared with the baseline, and the average score of Visit 3 decreased by 27.44±15.94 points compared with the baseline. The mixed effect model and paired rank test (Wilcoxon signed-rank test) analysis showed that both differences were statistically significant (p<0.05), indicating that the SAS scale score was significantly reduced compared with the baseline through treatment.

### 8.2 SDS scale (self-rating depression scale) score results

The results are shown in Tables 3 and 4.

**Table 3 Measured values of scale score**

| | **Visit 1** | **Visit 2** | **Visit 3** | **Statistics** | **P value** | **Test method** |
|---|---|---|---|---|---|---|
| SDS scale total score | | | | | | Mixed effect model |
| N (Nmiss) | 304 (1) | 305 (0) | 303 (2) | 528.56 | <.0001 | |
| Mean±SD | 61.3±13.82 | 43.74±14.4 | 32.99±11.33 | Visit 1VS2 | <.0001 | |
| Median (Q1, Q3) | 55 (50, 70) | 44 (30, 54) | 28 (25, 36) | Visit 1VS3 | <.0001 | |
| Min, Max | 36, 100 | 25, 86 | 25, 66 | Visit 2VS3 | <.0001 | |

**Table 4 Difference of SAS scale scores**

| | **Visit 2-Visit 1 difference** | **Visit 3-Visit 1 difference** |
|---|---|---|
| The difference between the total SAS score after treatment and the baseline | | |
| N (Nmiss) | 304 (1) | 303 (2) |
| Mean±SD | -17.63±11.56 | -28.34±15.58 |
| Median (Q1, Q3) | -19 (-25, -10) | -26 (-36, -20) |
| Min, Max | -50, 17 | -75, 11 |
| Statistics | -21357.5 | -22479.5 |
| P value | <0.0001 | <0.0001 |
| Test method | paired rank test | paired rank test |

Through intervention treatment, the average score of Visit 2 during the study decreased by 17.63±11.56 points compared with the baseline, and the average score of Visit 3 decreased by 28.34±15.58 points compared with the baseline. The mixed effect model and paired rank test analysis showed that the differences were statistically significant (p<0.05), indicating that the SDS scale score was significantly reduced compared with the baseline through treatment.

### 9. Conclusion

The Shenling Baizhu can treat neuropsychiatric symptoms such as anxiety, depression, and insomnia of recovered COVID-19 patients.

### Industrial Applicability

The present application provides use of Shenling Baizhu in the preparation of a medicine for treating neuropsychiatric symptoms of recovered COVID-19 patients; wherein the raw material composition of the Shenling Baizhu comprises: ginseng, *rhizoma atractylodis* macrocephalae stir-fried with bran, poria cocos, Chinese yam, lotus seed, fried white hyacinth beans, coix seed, fructus amomi, platycodon grandiflorus, and liquorice The applicant has found that the Shenling Baizhu can treat neuropsychiatric symptoms such as anxiety, depression, and insomnia of recovered COVID-19 patients, and therefore has excellent industrial applicability.

## Claims

1. Use of Shenling Baizhu in the preparation of a medicine for treating neuropsychiatric symptoms of recovered COVID-19 patients, wherein the raw material composition of the Shenling Baizhu comprises: ginseng, *rhizoma atractylodis macrocephalae* stir-fried with bran, poria cocos, Chinese yam, lotus seed, fried white hyacinth beans, coix seed, fructus amomi, platycodon grandiflorus, and liquorice.

2. The use according to claim 1, wherein the neuropsychiatric symptoms of recovered COVID-19 patients comprise at least one of insomnia, anxiety, and depression.

3. The use according to claim 1 or 2, wherein the medicine is a clinically acceptable formulation.

4. The use according to claim 3, wherein the medicine is selected from a soup, a pill, a powder, a granule, a tablet, or a capsule.
